(19) Europäisches Patentamt
European Patent Office
Office européen des brevets

(11) EP 3 904 492 A1

(12) EUROPEAN PATENT APPLICATION

(43) Date of publication:
03.11.2021 Bulletin 2021/44

(51) Int Cl.:
*C12M 1/00* (2006.01)

(21) Application number: 20172130.5

(22) Date of filing: 29.04.2020

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(71) Applicants:
• Nofima AS
9019 Tromsø (NO)
• ELEA Vertriebs-und Vermarktungsgesellschaft mbH
49610 Quakenbrück (DE)

(72) Inventors:
• FERNÁNDEZ, Estefanía Noriega
4005 Stavanger (NO)
• PARNIAKOV, Oleksii
49610 Quakenbrück (DE)

(74) Representative: Grünecker Patent- und Rechtsanwälte
PartG mbB
Leopoldstraße 4
80802 München (DE)

(54) METHOD AND SYSTEM OF PRE-TREATING BIOMASS, IN PARTICULAR BIOMASS THAT IS RESISTANT TO CELL DISRUPTION, FOR IMPROVING THE ACCESSIBILITY OF CELLULAR COMPOUNDS THEREFROM

(57) The invention relates to a method and a system (1) of pre-treating biomass (2), in particular biomass (2) that is resistant to cell disruption, for improving the accessibility of a compound therefrom. The inventions further relates to a method for extracting a compound from a biomass (2), in particular a biomass (2) that is resistant to cell disruption, comprising the steps of pre-treating the biomass (2) and extracting the compound from the pre-treated biomass (2). In order to improve the extraction of compounds from biomass (2) that is resistant to cell disruption, the inventive method comprises the step of incubating the biomass (2) in plasma activated water (4). The inventive system (1) comprises a plasma activated water stage (3) configured to incubate the biomass (2) in plasma activated water (4); and an electric field stage (5) comprising at least one capacitor (6) for generating and applying an electric field to the biomass (2).

FIG. 1

EP 3 904 492 A1

## Description

The present invention relates to a method of pre-treating biomass for improving the accessibility of compounds therefrom. The present invention in particular relates to a method of pre-treating biomass, in particular biomass that is resistant to cell disruption, for improving the accessibility and in particular, the extraction of compounds such as intracellular compounds therefrom.

The present invention further relates to a method for extracting a compound, in particular an intracellular compound, from a biomass, in particular a biomass that is resistant to cell disruption, comprising the steps of pretreating the biomass and extracting the compound from the pre-treated biomass.

The present application further related to a system for pre-treating biomass, in particular biomass that is resistant to cell disruption, for improving the accessibility of a compound therefrom.

Cell wall disruption is a critical upstream process for the extraction of valuable compounds from a biomass feedstock, such as microalgae, including compounds like lipids, proteins, pigments, vitamins and other bioactive compounds. Microalgae, like *Chlorella* spp. have been extensively commercialized over the past 40 years as food and feed supplements on account of their rapid growth rate and productivity, tolerance over a wide range of environmental conditions and high content in essential nutrients and protein. Algae provide a promising source for bioactive compounds, including pigment and protein availability for high value markets, such as cosmetics, pharmaceuticals, nutraceuticals, animal livestock and human nutrition.

However, the remarkably robust and thick cell wall of microalgal cells, such as *Chlorella* spp., is a major barrier for extraction of intracellular compounds. At the same time, cell wall disruption is the most determinant step for recovery of valuable compounds to ensure intensified mass transfer and efficient extraction.

A practical disruption strategy should require low energy input and low operating cost while providing high recovery and quality of the final product. Cell disruption methods can be categorized into mechanical (bead-beating, pressing, ultrasonication, autoclaving and homogenization) and non-mechanical methods (osmotic shock, chemicals, enzymes). Mechanical methods are the most suited methods on an industrial scale, but they also have the disadvantages of high energy, high pressure, and/or high temperature requirements. Such conventional disintegration methods, e.g. mechanical shearing or heat treatment with organic solvents, are time consuming and pose environmental and health risks, and may cause denaturation or transformation of valuable compounds, besides low extraction yields and low specificity for such a robust biomass like microalgae. Other methods like sonication have severe health and safety issues for operators due to noise and sonication generates heat that harms heat sensitive, thermo-labile materials. Chemical methods are risky to use and might affect the nature of the recovered product, as well as contribute to environmental footprint.

WO 2019/121630 A1 and WO 2017/081677 disclose methods for extracting molecules from algae, comprising the step of applying a pulsed electric field (PEF), which is a non-thermal disruption method. However, PEF is a rather ineffective disruption method for biomass holding rigid cell walls, such as e.g. microalgae like *Chlorella sorokiniana.*

Thus, there is a need on the market for a safe, eco-friendly, mild and cost-efficient technique to improve the accessibility of compounds from a biomass and/or for the accessibility of sensitive compounds that are decomposed when exposed to heat or chemicals encountered in conventional cell disruption process.

The objective technical problem to be solved by the present invention is thus considered to improve the accessibility of compounds from biomass in general and in particular, to enable accessibility from biomass that is resistant to cell disruption and/or to enable accessibility of sensitive compounds that easily decompose.

The present invention solves this problem by providing a method of pre-treating biomass, in particular biomass that is resistant to cell disruption, for improving the accessibility of compounds, in particular intracellular compounds therefrom, comprising the step of incubating the biomass in plasma activated water.

The system of the present invention solves this problem by comprising: a plasma activated water stage configured to incubate the biomass in plasma activate water; and an electric field stage comprising at least one capacitor for generating and applying an electric field to the biomass. In particular, the system of the invention is configured for conducting the method of the present invention.

Surprisingly, incubating the biomass in plasma activated water (PAW) disrupts the cell walls and cell membranes of the biomass facilitating the release of substances, in particular intra-cellular substances, improving mass transfer and achieving a faster penetration of solvents into the cell and hence improving the extraction of compounds from the biomass. The expressions "substance" and "compound" are used synonymously. So, target compounds from biomass become accessible due to the present inventions that would otherwise not be or just poorly released from the biomass. This is insofar astonishing as the pre-treatment method of the present invention is even effective on biomass that is resistant to cell disruption, and on which other disruption methods are rather ineffective. The inventive method is not limited to improve accessibility of compounds from biomass that is resistant. The invention likewise improves posttreatment process, such as a digestion or extraction of the pre-treated biomass by reducing the time required for downstream process step due to the improved accessibility. The inventive solution furthermore provides a mild and cost-effective pre-treatment of biomass that makes sensitive compounds from any kind of biomass accessible because it does not harm unstable compounds, which decompose in the conventional disruption processes using e.g. high pressures or temperatures or harsh chemicals. Further, the inventive method surprisingly allows for a controlled and selective disruption or permeabilization of the cell membrane, with which does not destroy the cells. This is advantageous because difficulties arise from a non-controlled cell destruction that results from an unhindered release of all intracellular products. Due to the controlled and selective permeabilization, only specific compounds are made accessible, such as for example only compounds from a certain cell organelle and/or compounds having a certain size.

Improving accessibility in the sense of this invention in particular refers to improving the extraction thereof. However, improving accessibility is not limited to improving extraction and also refers to making a compound susceptible to digestion or removal that, without the incubation in PAW, is not available. For example, improved accessibility also encompasses making an initially indigestible compound from biomass digestible for an organism by incubation in PAW. Whether the accessibility improves can be determined by comparing the release (e.g. extraction, digestion) in a control without an incubation in PAW with the release according to the invention including the step of incubating the biomass in plasma activated water.

Cells and biomass that is resistant to cell disruption in the sense of this application refers to biomass from which a target compound cannot be extracted or can be extracted only under poor extraction yield due to e.g. taxonomic and phylogenetic diversity (bacteria, fungus, yeast, algae, etc.), structure and composition of the cell wall/membrane (e.g. fungal and Gram-positive bacteria cells are more resistant than Gram-negative bacterial cells), physiological status of the target organism (e.g. bacteria are more resistant at the stationary phase of growth than at the exponential phase of growth), accessibility (where the compound is located, if it is bound to cell components), and efficacy of the extraction method and operational conditions (e.g. non-hydrosoluble, ultrasound, bead-milling). Poor extraction yield can for example be an extraction of below 50%, below 40%, below 30%, below 20%, below 10%, below 5% or below 1% of the total content of said compound in the biomass. Poor extraction yield may in particular be considered a concentration close to or below the detection limit of the standard analytical method for quantification of the compound. Such biomass resistant to cell disruption in particular comprises cells and biomass that is not disrupted by a standard cell disruption procedure such as for example ultra-sonication (e.g. 54 kJ in 10 min) or beat-beating (e.g. 10 min at 10 Hz with 150 mg of 0.1 mm glass beads in 2 mL liquid). In a preferred embodiment, cells and biomass that is resistant to cell disruption in the sense of this application is disrupted neither by the standard procedure of ultra-sonication nor by the standard procedure of beat-beating.

Plasma activated water is produced using water, gas and electricity. For instance, ambient air is ionized into the plasma phase by applying a high voltage electrical field. The activation of water through its exposure to a gas plasma discharge results in the generation of reactive oxygen and nitrogen species (RONS) causing an increase in the redox potential and electrical conductivity and often affecting the pH of the media (acidification). While plasma activated water is known to have disinfecting properties, it was surprisingly found that it can be effectively used to pre-treat and disrupt biomass, in particular biomass that is resistant to cell lysis with other methods, in a manner improving the accessibility of compounds from the pre-treated biomass.

The above solution may be further improved by adding one or more of the following optional features. Each of the following features is advantageous on its own and may be combined independently with any other optional features.

According to one embodiment, the biomass can be incubated for at least 5 minutes, preferably 10 to 120 minutes, more preferably 30 to 90 minutes and most preferably 45 to 75 minutes. Within this time frame, an effective and controlled permeabilization of the cell wall and membrane is achieved, while incubating the biomass in the plasma activated water. This rather short time frame even allows a method, in which the biomass is continuously processed within an incubation device having a residence time of the biomass of at least 5 minutes, preferably 10 to 120 minutes, more preferably 30 to 90 minutes, and most preferably between 45 and 90 minutes.

In another embodiment, the plasma activated water can have an acidic pH value of lower than 3.5, preferably lower than 3.0, and most preferably lower than 2.5. This low pH value contributes to the cell disruption of the resistant biomass, even though, as will be explained later in detail, the low pH is only one of the attributes of plasma activated water resulting in the effective pre-treatment.

According to another embodiment, the plasma activated water has a high oxidation reduction potential of over 250 millivolts (mV), preferably above 350 mV, and most preferably above 450 mV. This high oxidation reduction potential contributes to damaging the cell membranes and cell walls. It allows to selectively permeabilize cell from biomass in a mild manner that does not harm sensitive compounds, and affects even robust biomass, thus improving the extraction of cytoplasmic and other intra-cellular compounds The terms "biocompound" and "compound from a biomass" are used synonymously and can be interchanged.

In another embodiment, the plasma activated water comprises a nitrate concentration of at least 40 milligrams per liter (mg/L), preferably of at least 150 mg/L, and most preferably of over 350 mg/L. The plasma activated water may alternatively or additionally comprise a nitrite concentration of at least 2 mg/l, preferably of over 6 mg/L and most preferably over 12 mg/L. This reactive nitrogen species (RNS), commonly used in food preservation, in the plasma activated water also exert a stress mechanism on the biomass cells, damaging the structure of even robust cells.

The plasma activated water may comprise reactive oxygen species (ROS), such as for example hydrogen peroxide, in a concentration of preferably above 20 $\mu$M, more preferably above 50 $\mu$M, and most preferably above 100 $\mu$M, and even more preferably of above 0.6 mM or higher. Hydrogen peroxide is one of the persistent species in the PAW that is commonly associated to its antimicrobial activity.

In a further embodiment, the method comprises a further pre-treatment step of applying an electric field to the biomass. It was surprisingly discovered that a synergistic effect can be observed when pre-treating cells, in particular cells that are resistant to cell disruption, by a combination of an incubation in plasma activated water and the application of an electric field. These two pre-treatment steps seem to affect certain cell structures differently, thus a combination of incubation with PAW and the application of an electric field makes compounds from biomass accessible that otherwise would not be digestible or extractable. Both pre-treatment steps can affect the cell structure selectively, such as only partially (e.g. just destroy the cell membrane while leaving the cell wall intact) and/or just temporarily (e.g. a reversible permeabilization is possible applying an electric field, such as a pulsed electric field). This facilitates the access to or extraction of certain compounds significantly because only a part of the cell components is released.

The applied electric field can in particular be a non-thermal electric field, in which the upper energy limit is determined in such a way that essentially no heating in the sense of ohmic heating takes place making the step advantageous for improving the accessibility and extraction of heat-sensitive compounds.

In a further embodiment, a pulsed electric field may be applied to the biomass. The electric field, in particular the electric pulses, can be generated both by direct contact of electrodes connected with a pulse generator for generating a high voltage between the electrodes, as well as indirectly by way of conductive fluids, in which the biomass is totally or in part submerged. A high voltage pulse generator generating electric fields in the form of short pulses in the micro- to millisecond range of a high voltage in the kilovolt range can be used for generating pulsed electric fields. Such high voltage pulses may cause electroporation in the biomass, in particular, permeabilizing the cell membrane, producing pores in the cell membrane. In terms of time and energy optimization, the biomass can be treated with at least 10 electric pulses, preferably 10 to 300 electric pulses, and most preferably 40 to 80 electric pulses. One further advantage of applying an electric field is that this is a fast process lasting less than a minute, can continuously process the biomass and can be used to improve the accessibility and extraction of temperature sensitive biocompounds.

When applying the electric field, an energy input of at least 10 kilojoules per kilogram (kJ/kg), preferably at least 50 kJ/kg, and most preferably at least 100 kJ/kg can be applied to the biomass. Energy input of this magnitude is particular suited to effectively electroporate the biomass, i.e. permeabilize the cell membranes. Applying an energy input in the range of 50 to 200 kJ/kg achieves the desired electroporation to biomass that is susceptible to this disruption method, while at the same time avoiding unnecessary over-processing, wasting time and energy.

It has been observed that it is advantageous if an electric field of 1 to 50 kilovolts per centimeter (kV/cm), preferably of 5 to 20 kV/cm, and most preferably 8 to 12 kV/cm is applied. Such field strengths can be obtained with commercially available industrial capacitors and voltage generators, such as a Marx generator, and prevent unwanted thermal effects from occurring, which could lead to undesired chemical modification of temperature sensitive biocompounds.

According to a further embodiment, the electric field can be applied before, while and/or after the step of incubating the biomass in plasma activated water.

In one embodiment, the biomass is first incubated in plasma activated water and subsequently, after the incubation, an electric field is applied. Preferably, the electric field is applied to biomass in the plasma activated water.

The method for extracting a compound from a biomass, in particular a biomass that is resistant to cell disruption, comprises the steps of pre-treating the biomass according to the present invention, and extracting the compound from the pre-treated biomass.

The biomass pre-treated and/or used in the extraction method may be any cell and tissue from human, animal, plant and microbial sources. Microbial sources include inter alia yeast, fungus, bacteria and spores. The biomass may in particular comprise algae, such as macroalgae and/or microalgae, animal and/or plant-based raw material like non-edible seeds, a waste product, such as for example, fermentation residues, and waste products from food production.

The biocompound to be made accessibility and/or to be extracted may be a lipid, such as a polyunsaturated fatty acid like eicosapentaenoic acid or docosahexaenoic acid, a protein, a carbohydrate, a vitamin such as tocopherols, a phenolic compound, intracellular DNA/RNA, an organelle, an enzyme, or a cell wall compound like Glucans, e.g. $\beta$-(1,3)/(1,6)-Glucans, as well as a pigment like chlorophyll, phycocyanin and carotenoids, $\beta$-carotene, astaxanthin, zeaxanthin, sitosterol, microcolin, lutein, and phycobiliproteins.

In one embodiment, the systems according to the invention may comprise at least two electrodes connected to a pulse generator. The electric field, especially the electric pulses, can be generated by direct contact of the capacitor or its electrodes with the biomass, as well as by conductive fluids. In one embodiment, the conductive fluid is plasma activated water with the incubated biomass coming from the plasma activated water stage. Various electrode shapes can be used, such as plate, ring, grid, hollow or flow-through electrodes. A high-voltage pulse generator can be used as a pulse generator, which generates electric fields in the form of short pulses in the micro to millisecond range of a high voltage in the kilovolt range. Such high-voltage pulses cause electroporation in the biomass, which in particular results in a simple and non-thermal permeabilization of the cell membrane.

In a further embodiment, the system may comprise a transport device configured to move the biomass through stages of the system. The transport device may be a pump, a transport belt or a transport screw, depending on the biomass to be pre-treated.

In one embodiment, the plasma activated water stage comprise an incubation container and a plasma water generation device for producing plasma activated water. The incubation container may be an incubation tank or an incubation piping. The incubation container may comprise a mixer for agitating the biomass in the incubation container.

In a further embodiment, the system may further comprise a temperature control system. The temperature control system may be configured to control the temperature of the plasma activated water. It may for example comprise a heating and cooling device to raise and/or lower the temperature of the plasma activated water. The temperature control system may further comprise a temperature sensor for measuring the temperature of the plasma activated water. A temperature control system with a heating and cooling device as well as a temperature sensor may be configured to adjust the temperature of the plasma activated water within a set range, e.g. keep it below a determined threshold temperature. This way it can be excluded that the temperature increasing too much and the plasma activated water or a thermolabile target compound decomposes. The temperature control system may be a closed-loop (or feedback control) system with a heating and cooling device, a temperature sensor and a controller having a pre-set reference temperature.

In the following, the invention will be described by way of example, in detail, with reference to the drawings and subsequent experimental examples using advantageous embodiments. The advantageous further developments and configurations illustrated are independent from each other and can be combined with one another at random, depending on the requirements of the application.

In the Figures:

Fig. 1: shows a schematic overview of the experiments performed for conducting an exemplary embodiment of a method of pre-treating a biomass, in particular a biomass that is resistant to cell disruption, for improving the accessibility of a compound therefrom, as well as a method for extracting the compound from the biomass;

Fig. 2: is a graph showing the pH condition of plasma activated water;

Fig. 3: is a graph showing the temperature of the plasma activated water upon its production;

Fig. 4: is a graph showing the nitrate concentration in the plasma activated water produced;

Fig. 5: is a graph showing the nitrite concentration in the plasma activated water produced;

Fig. 6: is a graph showing the stability of nitrites in plasma activated water after 2 week storage at 4°C;

Fig. 7: is a graph showing the pH stability in plasma activated water after 2 week storage at 4°C;

Fig. 8: is a graph showing the nitrate stability in plasma activated water after 2 week storage at 4°C;

Fig. 9: is a graph showing the protein extraction achieved using the method of the present embodiment;

Fig. 10: is a graph showing the chlorophyll extraction of the method according to the exemplary embodiment;

Fig. 11: is a graph showing the proportions of water-soluble proteins after PEF treatment;

Fig. 12: is a schematic diagram showing an embodiment of a system for pre-treating biomass; and

Fig. 13: is a schematic diagram showing another embodiment of a system for pre-treating biomass.

Experiments

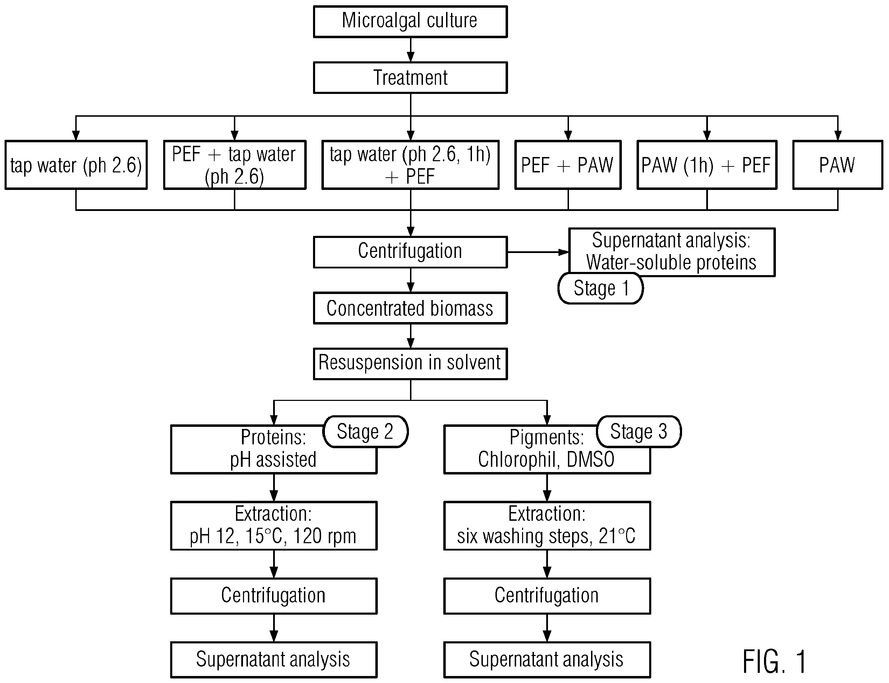

Fig. 1 shows an overview of the experiment conducted for an exemplary method of pre-treating a biomass, in the experiment a microalgae, for improving the extraction of a compound therefrom. In the experiment conducted, the microalgae culture sample is treated as follows:

A. with tap water having an adjusted pH of 2.6, serving as a control;

B. with the tap water of the control that was treated with a pulsed electric field (PEF).

C. the sample was incubated in the tap water of the control for 1 hour and subsequently treated with PEF;

D. the sample was first treated with PEF, followed by incubation in plasma activated water for 1 hour;

E. the sample was first incubated in plasma activated water for 1 hour and subsequently treated with PEF; and

F. the sample that was incubated in plasma activated water for 1 hour only (no PEF).

Subsequent to the treatment, the sample was centrifuged and the water-soluble proteins from the supernatant (stage 1 in Fig. 1) were used for further analysis. The concentrated biomass resulting from the centrifugation was re-suspended in a solvent and the proteins and pigments, namely chlorophyll, were extracted and analyzed in the re-suspension.

## 1. Biomass/Microalgae Culture

Microalgae was used as biomass feedstock. *Chlorella sorokiniana* was obtained from AlgaeHolland B.V. (Vlissingen, Zeeland, Netherlands). The cultivation took place in raceway ponds at 15 - 22 °C.
After reaching cell dry weight (cdw) of 0.8 - 1.0 g/100g, microalgal biomass was harvested, concentrated by filtration up to cdw = 15 g/100g and shipped within one day in 10 or 20 L buckets as fresh microalgae suspensions for analysis. Depending on the interval between harvest and processing (shipment time and utilisation of the pilot plant), suspensions showed pH 5.3 - 5.6.
The dry weight of the algae was measured to adjust the weighing for the extraction procedures. Therefor around 5 g of fresh algal suspension were applied on the aluminum pan of the moisture analyzer VPB-10 (Henk Maas Weegschalen B.V., Veen, Netherlands) and dried at 105 °C until reaching a constant dry matter. Conductivity of microalgal suspension was measured by the conductivity meter Cond 3310 (conductivity cell: Tetra-Con 325; Xylem Analytics Germany GmbH, Weilheim, Germany) at room temperature (21 °C).

## 2. Cell Disruption

## 2.1.Pulsed Electric Field (PEF) Treatment

PEF treatments of microalgae were carried out with two different systems: the trial batch system Cell Crack II and the continuous 5 kW pilot scale PEF system HVP 5 v1.32 (Elea Vertriebs- und Vermarktungsgesellschaft mbH, Quakenbrück, Germany). Characteristics of both systems are shown in the Table below:

| Parameter | HVP 5 | Cell Crack II |
|---|---|---|
| Mode of operation | Continuous | Batchwise |
| Maximum voltage | 30 kV | 30 kV |
| Pulse width | 4 - 32 μs | 5 - 50 μs |
| Pulse frequency | 1 - 1000 Hz | 2 Hz |
| Capacity | 30 - 200 L/h | 0.2 - 10 L |
| Electrode gap | 1 cm | 2 - 28 cm |

By defining the charging voltage U [kV] and electrode gap d [cm], the electrical field strength E [kV/cm] was set:

$$E = \frac{U}{d}$$

The specific field strength Ws [kJ/kg] characterizes the specific energy input and is one of the main parameter and uses the energy per pulse (Wp), the mass flow and the frequency for calculation:

$$W_S = \frac{W_p * f}{\dot{m}}$$

For batch treatment, the term f/m can be replaced by n/m, where n is the pulse number and m the mass of the sample in the treatment chamber.
The experimental PEF conditions were: E = 10 kV/cm; Ws = 150 kJ/kg.

## 2.2. Plasma activated water (PAW)

## 2.2.1. PAW Generation and Composition

A cold plasma (CP) reactor, consisting of the powered and ground electrodes and a 1 mm thick quartz disc between them, was set up to generate a surface barrier discharge (SBD). Such a configuration was coupled to the lid of the treatment chamber (176 x 174 x 48 mm), with a total discharge area of 15 cm2. Since species with high oxidation potential are also short-lived, the gap distance between the SBD and the liquid (remote generation mode) limits the flux reaching the water surface and thus, efficiency drops. However, placing electrodes in contact with the liquid can also lead to further contamination and accelerated electrode wear. For 100 mL treatment volume, the gap distance between the liquid surface and the electrode was 44.8 mm (3.2 mm water column). The CP generating source (Oscilloscope TENMA, 2 Channel, 200 MHz, 2 GSPS, 16 Mpts; Function Generator AIM-TTI INSTRUMENTS, Direct Digital Synthesis (DDS); High-voltage power supply; High-voltage probe TEKTRONIX, 75 MHz, 20 kV, 1000:1) produced a sinusoidal signal at a frequency of 12 kHz. Plasma power was determined using the mean of the product between the applied voltage and current over 200 cycles. The system operated at atmospheric pressure, with room air as the plasma-inducing gas.

CP activation experiments were conducted with tap water under standard/constant conditions of magnetic stirring (500 rpm), initial volume (100 mL), pH (no adjustment, ≈5.3-6.0) and temperature (room temperature, ≈19-21 °C). A plasma power of 16, 26, and 36 W, corresponding to a voltage peak-to-peak value of 9, 10 and 11 kV, respectively, and exposure time of 5, 12.5, and 20 min was applied.

pH and oxidation reduction potential (Mettler Toledo SevenGo Pro pH/ion meter, Mettler Toledo, Oslo, Norway) and immediate temperature reading (Raytek MiniTemp FS Infrared Food Thermometer, Raytek, Oslo, Norway) were determined in PAW samples.

Spectroquant® test kits (Merck, Oslo, Norway) were used for spectrophotometric determination (Shimadzu UVmini-1240-UV-VIS, Shimadzu, Tokyo, Japan) of nitrates (#109713; analogous to DIN 38405-9) and nitrites (#114776: analogous to EPA 354.1, APHA 4500-NO2-B, and DIN EN 26 777) at 340 and 525 nm, respectively. Hydrogen peroxide was determined with the titanium sulphate colorimetric method at 407 nm. As nitrites may interfere with the determination of hydrogen peroxide due to the acidic PAW environment, the protocol was slightly modified by adding sodium azide 60 mM to the acidic samples prior mixing with the titanium sulfate reagent, to scavenge reactive species (nitrites are reduced into molecular nitrogen) that could otherwise react with hydrogen peroxide. Calibration curves for nitrates (0.5-100.0 mg/L), nitrites (0.01-3.00 mg/L) and hydrogen peroxide (0.1-5.0 mM) were determined in triplicate on independent days with distilled water as blank.

After exposure to CP and prior to the quantitative determination of reactive species, PAW samples were tempered at 15-20 °C, which is the optimal range for analytical determination, and appropriate dilutions were prepared with distilled water at room temperature.

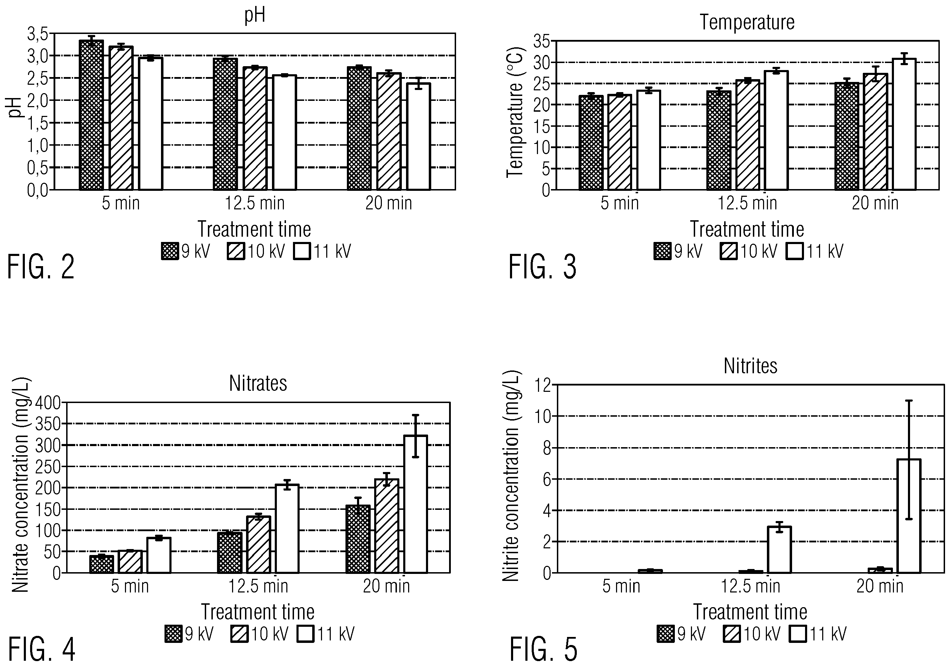

As can be seen in Figures 2 to 5, the plasma activated water has a pH of below 3.5, preferably below 3.0 and most preferably below 2.5. The temperature did not significantly increase and a significant amount of reactive nitrogen species was produced. The concentration of nitrites was above 2 mg/L, preferable above 6 mg/l. The concentration of nitrates was above 40 mg/L, preferable above 100 mg/l and most preferably above 200 mg/L.

In order to evaluate the stability of reactive species in the PAW, PAW samples produced in a 20 min treatment were stored at 4 °C for 24 h, for 7 days and for 14 days.

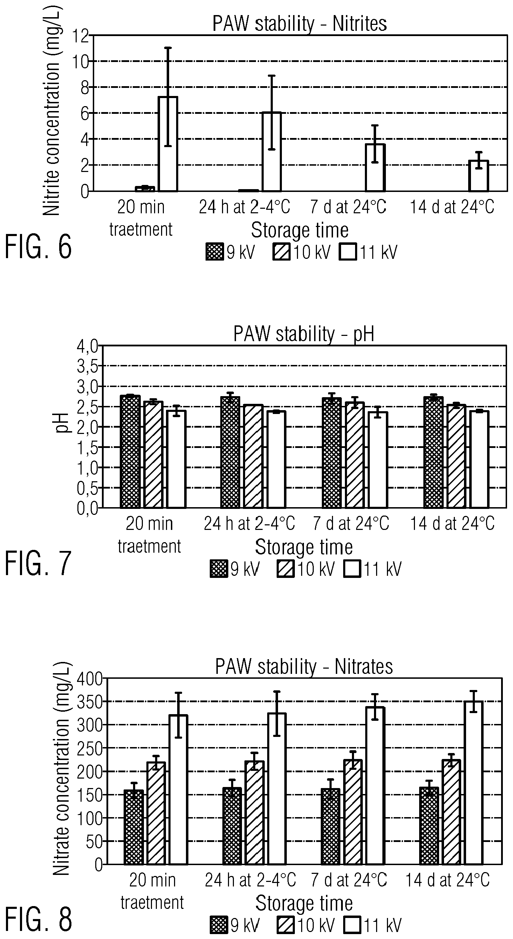

As can be seen in Figures 6 to 8, the plasma activated water maintained its pH, nitrite and nitrate concentration during storage.

### 2.2.2. PAW treatment

For the PAW treatment, the biomass sample was incubated in the PAW for 1 hour at room temperature.

As a control, tap water with an adjusted pH of 2.6 was used.

### 3. Extraction Procedures

### 3.1. Protein Extraction

Protein extractability of the microalgae suspensions was investigated at two different points of processing. First, around 2 h after PEF treatment to examine the yield of water-soluble proteins after electroporation of the cells (stage 1 in Fig. 1), and second after resuspension of centrifuged biomass in tap water at initial concentration (cdw = 1 g/100g) and pH adjustment to increase protein solubility (stage 2 in Fig. 1). pH, measured by the pH meter SI Analytics Lab 865 (Xylem Analytics Germany GmbH, Weilheim, Germany) was adjusted with 2 M NaOH in the range of 11.9 - 12.0. Samples in 15 mL Falcon tubes were placed into a shaking incubator (120 rpm, 15 °C,) for 1 hour and centrifuged subsequently (4500 xg; 5 min) to obtain cell-free extracts.

Determination of the protein content was conducted with a bicinchoninic acid (BCA) protein assay kit (Thermo Fisher Scientific Inc., Waltham, MA USA). For this purpose, 0.1 mL of the extract were mixed with 2.0 mL working reagent. After 2 h incubation at room temperature (21 °C), absorbance A [AU] of the samples was measured by using the spectrometer GeneQuant 1300 (Biochrome US, Holliston, MA USA) at $\lambda$ = 562 nm. Calibration of the method took place by preparation of a kit including BSA standards.

### 3.2. Pigment Extraction

Pigment extraction was realised by washing of microalgae with dimethyl sulfoxide (DMSO, 99.9%+, Thermo Fisher GmbH, Kandel, Germany). Next to the application of pure solvents (100 mL/100 mL) a binary mixture with water holding 70 mL/100 mL DMSO was used for extraction (Parniakov et al., "Pulsed electric field assisted extraction of nutritionally valuable compounds from microalgae Nannochloropsis spp. using the binary mixture of organic solvents and water", Innovative Food Science and Emerging Technologies, 2015, Vol. 27, pages 79-85). Concentrated biomass was resuspended in organic solvent (1 or 15 g dry matter/100 mL extraction solvent) and washed six times to extract the majority of combined chlorophylls. Each washing step was conducted by the addition of 10 mL solvent into 15 mL falcon tubes, resuspension by means of a stirring rod, vortexing for 60 s and immediate centrifugation (4500 xg, 5 minutes) at ambient temperature (21 °C).

The pigment content of the extract was analyzed with the GeneQuant 1300 spectrometer (accuracy: $\pm$ 2 nm; Biochrome US, Holliston, MA USA) within the absorbance range of $\lambda$=200 - 700 nm. Values for chlorophyll a ($A_{Cha}$) at $\lambda$=665 nm and chlorophyll b ($A_{chb}$) at $\lambda$=649 nm were taken subsequently. Pigment concentrations of chlorophyll a ($Ch_A$), chlorophyll b ($Ch_B$), combined chlorophylls ($Ch_C$= $Ch_A$ + $Ch_B$) were calculated according to following equations:

$$Ch_A = 12{,}47 * A_{Cha} - 3{,}62 * A_{Chb}$$

$$Ch_B = 25{,}06 * A_{Chb} - 2{,}43 * A_{Cha}$$

### 4. Results

#### 4.1. Effect of PEF Treatment (Samples b and c)

##### 4.1.1. Protein Content and Yield

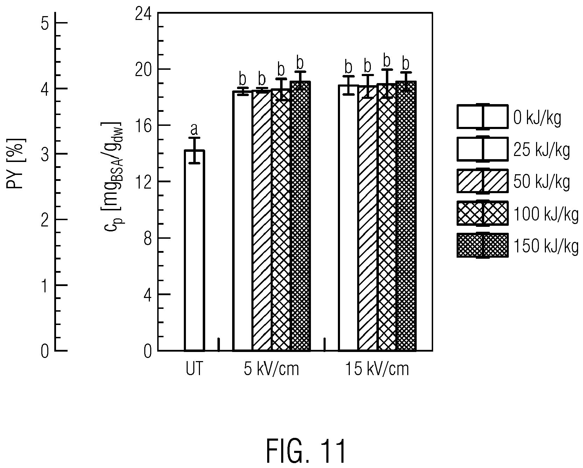

Proportions of water-soluble proteins after PEF treatment of fresh *Chlorella sorokiniana* suspensions at $T_{in}$ = 20 °C are shown in Figure 11. Protein content Cp and corresponding protein yields *PY* of water-soluble proteins of *Chlorella sorokiniana* suspensions ($C_{dw}$ = ~ 15 g/100 g) for untreated (UT) and PEF treated at E = 5, 15 kV/cm and different specific energy intakes, $W_{specific}$ = 25, 50, 100 and 150 kJ/kg; input temperature $T_{in}$ = 20 °C; different letters between the classes indicate significant differences of mean values (n = 3; $\alpha$ = 0.05). In general, PEF treatment of algae led to an enhanced release of proteins compared to the control (UT, 0 kJ/kg) sample. After application of different electrical field strengths (E = 5 and 15 kV/cm), protein concentration increased by ~ 4 $mg_{BSA}/g_{dw}$ which corresponds to ~ 1 % of the total protein. Although a slight trend of increased protein yields at higher *E* and $W_{specific}$ was observed.

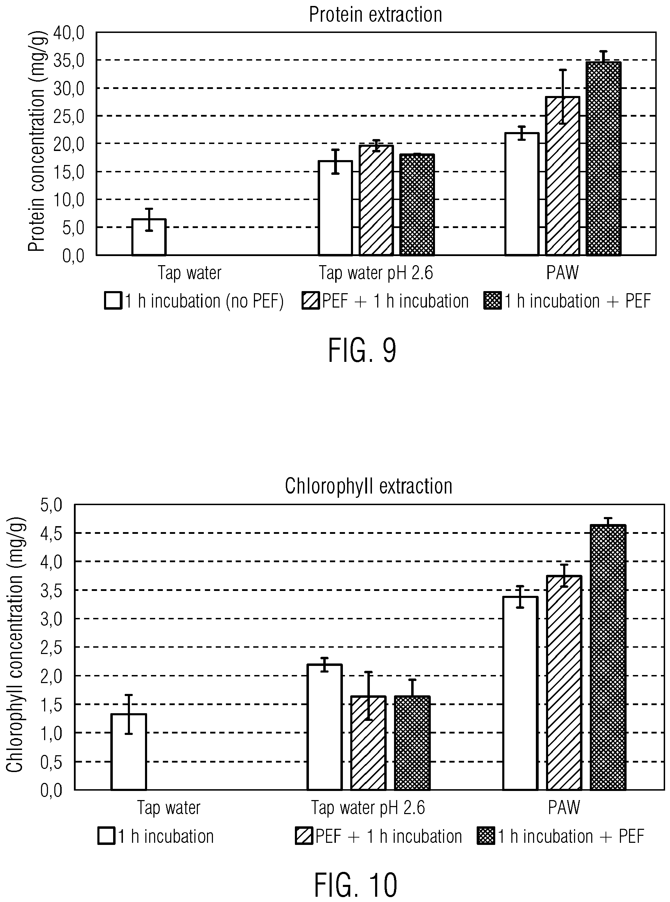

Liberation of proteins by the application of PEF is attributed to the electro-permeabilisation of the plasma membrane. The vacuole membranes are not affected, which results in a selective release of cytoplasmic proteins. The protein yields obtained for the PEF only samples are low compared to the protein extraction in the PAW sample and the combined PAW+PEF sample (see Fig. 9).

##### 4.1.2. Pigment Content

In general extraction of algal pigments from C. *sorokiniana* suspensions (*Chlorella sorokiniana* resuspended ($c_{dw}$ = 15 g/100 g)) with pure DMSO required around 6 - 8 washing steps in binary mixtures of dimethyl sufoxide (DMSO) and water holding 100, 70, mL/100 mL DMSO; extraction conditions within each washing step: 30 min; 160 rpm; 36 °C. By decreasing the solvent concentration, extractability of chlorophylls also declined. Peak washing steps, with extraction maxima, were observed at the second, third and fifth washing step for 100 and 70 mL/100 mL DMSO, respectively (no data shown). At this point chlorophyll extraction efficiency was rather low and around 33% for 70 mL/100 mL DMSO compared to the pure solvent.

Here, lower extraction yields compared to long-time experiments are due to the deceleration of extraction kinetics by decreasing solvent activities which is in line with previous studies of (Parniakov et al., 2015, cited above) who investigated *Nannochloropsis spp.* resuspended (1 g/100 g) in DMSO and EtOH. While they observed an almost instant extraction of pigments in 100 mL/100 mL DMSO, PEF pre-treatment only showed an enhanced pigment extraction using lower concentrations (50 mL/100 mL) of DMSO and EtOH.

Higher initial water proportions and agglomeration were assumed to be the major factors causing deceleration of pigment extraction in this study. Nevertheless, reducing solvent activity turned out to be less relevant for industrial application, as solvent effectiveness is quite low, and PEF was not able to enhance (long-time) or accelerate (short-time) extraction to an economical efficient level. Basically the PEF alone is not an efficient cell wall disruption method for extraction of biocompounds from microalgae with robust cell wall (tested here: *Chlorella*).

#### 4.2. Effect of PAW Treatment (Sample f)

##### 4.2.1. Protein Content and Yield

While PEF does not seem to improve the protein extraction, the protein content in the PAW-treated sample significantly increased about 3-times up to 7-times compared to a tap water control, as can be seen from Figure 9.

##### 4.2.2. Pigment Content

While PEF does not seem to improve the Chlorophyll extraction, the Chlorophyll content in the PAW-treated sample significantly increased about 3-times up to 5-times compared to a tap water control, as can be seen from Figure 10.

Contrary to PEF, PAW showed significantly better results than PEF and improves the extraction of compounds from a biomass that is resistant to cell disruption (tested here: *Chlorella*).

Thus, PAW has been demonstrated to significantly enhance the extraction of bioactives (proteins and pigments) from *Chlorella sorokiniana,* which is attributed not only to the low pH of PAW (controls in water at the same acidic pH yielded significantly lower bioactive levels) but also to the cocktail of reactive oxygen and nitrogen species (RONS) in the PAW, leading to high oxidation reduction potential (ORP). PAW stress mechanisms on bacterial cells have been associated to oxidative damage of the cell membrane, cell wall breakdown, cell shrinkage and cytoplasmic leakage, among others.

Thus, PAW disruption of the microalgal cell wall would lead to improved mass transfer, faster penetration of solvents into the cell and eventually, enhanced release of intracellular bioactives.

### 4.3. Combined Effect of PEF and PAW (Samples d and e)

Surprisingly, a synergistic effect of PAW and PEF has been observed, with 1 hour PAW incubation followed by PEF (10 kV/cm, 150 kJ/kg) yielding chlorophyll and protein concentrations three- and seven-fold higher, respectively, than the corresponding values of control samples (water at pH 2.6), as can be seen in Figures 9 and 10.

When combining PAW and PEF, the extraction could be further improved, even though PEF alone did not have an effect of extraction compound
The results show that the method of the invention has the potential for improving extraction of compounds from biomass that is resistant to cell disruption, such as microalgae that might normally not be considered for commercial application due to their thick and resistant cell walls.

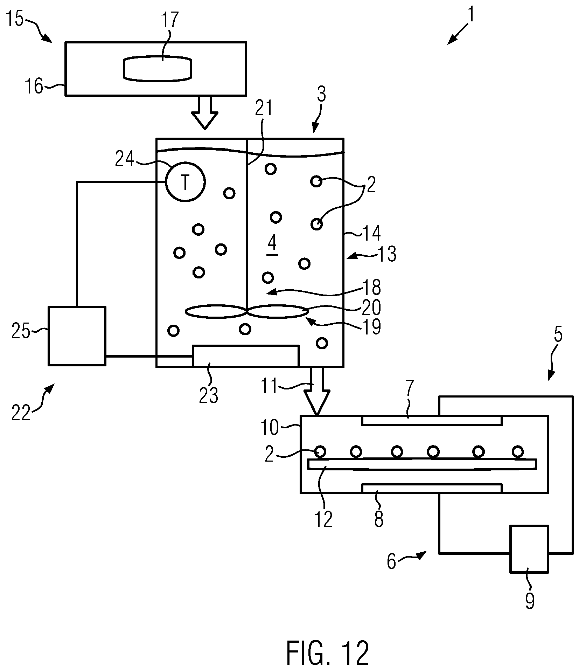

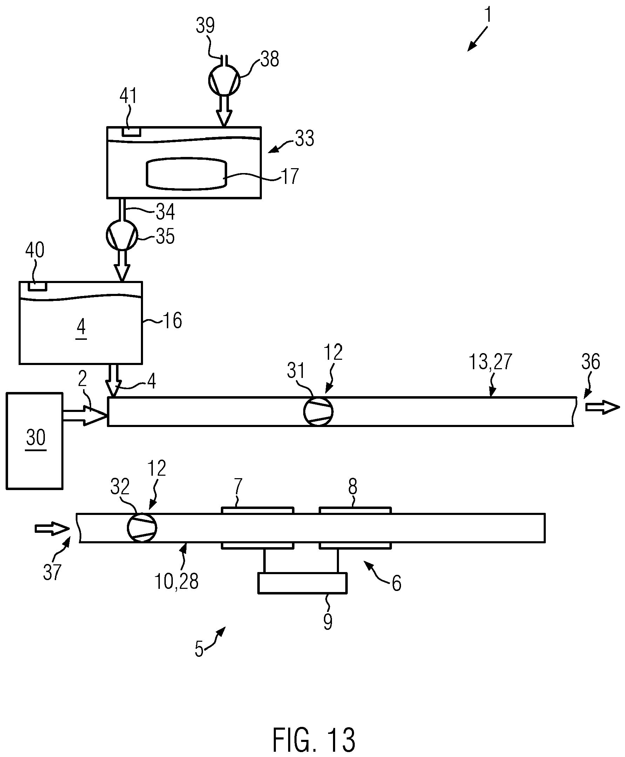

In the following, two exemplary embodiments of a system for pre-treating a biomass for improving the accessibility of a compound therefrom are described with reference to Figs. 12 and 13.

Fig. 12 shows a first exemplary embodiment of a system 1 for pre-treating biomass 2, such as for example biomass that is resistant to cell disruption, for improving the accessibility of a compound therefrom. The system 1 comprises a plasma activated water stage 3 that is configured to incubate the biomass 2 in plasma activated water 4, as well as an electric field stage 5 comprising at least one capacitor 6 for generating and applying an electric field to the biomass 2.

In the shown embodiment, the capacitor 6 comprises two electrodes 7,8 that are connected to a pulse generator 9, such as a Marx generator. The electric field stage 5 comprises an electroporation container 10 for receiving the biomass 2 in a conductive fluid. In the shown example, the conductive fluid is the plasma activated water 4 including the incubated biomass 2 that is transferred by a transfer element 11 from the plasma activated water stage 3 to the electric field stage 5. The electric field stage 5 furthermore comprises a transport device 12 for conveying the biomass 2 through the area between the two electrodes 7, 8, for applying an electric field to the biomass 2. In the shown embodiment, the transport device 12 is schematically depicted as a transport belt, on which the biomass 2 is received and transported through the electric field generated between the two electrodes 7 and 8. The transfer element 11 moving the biomass from the plasma activated water stage 3 to the electric field stage 5 may be considered another transport device that is configured to move the biomass 2 through the stages of the system 1.

In the embodiment shown in Fig. 12, the plasma activated water stage 3 comprises an incubation container 13. In the shown embodiment, the incubation container 13 is an incubation tank 14. In the incubation tank 14, the plasma activated water 4 is brought into contact with the biomass 2 and the biomass 2 is incubated for a predetermined disposal time in the plasma activated water 4. The plasma activated water 4 may be generated by a plasma activated water generation device 15. The plasma activated water generation device 15 is merely shown schematically. It comprises a plasma activated water container 16 and a plasma generator 17 included in the plasma activated water container 16. When water is placed into the plasma activated water container 16, and plasma is generated in the plasma activated water container 16 using the plasma generator 17, plasma activated water 4 is generated in the plasma activated water generation device 15. The generated plasma activated water 4 may either be directly transferred to the incubation container 13, as it is shown in the exemplary embodiment of Fig. 12. The plasma activated water 4 generated in the plasma activated water generation device 15 could likewise be stored in a storage device (not shown) and, when needed, be transferred to an incubation container 13, bringing it into contact with the biomass 2 to be pretreated therein.

The plasma activated water stage 3 further comprises a mixer 18 for agitating the biomass 2 in the incubation container 13. In the shown embodiment, the mixer comprises a stirrer 19, designed as an impeller 20 mounted inside the incubation tank 14 on a rotatable impeller shaft 21. The mixer 18 ensures an even distribution of the biomass 2 in the incubation container 13, thus improving the efficiency of the plasma activated water stage 3.

The plasma activated water stage 3, in the embodiment shown in Fig. 12, furthermore comprises a temperature control system 22 that is configured to control the temperature of the plasma activated water 4 in the incubation tank 14. The temperature control system 22 comprises a heating and cooling device 23 for raising and/or lowering the temperature of the plasma activated water 4 in the incubation tank 14. The temperature control system 22 furthermore comprises a temperature sensor 24 for measuring the temperature of the plasma activated water 4 in the incubation tank 14. In the shown embodiment, the temperature control system 22 furthermore comprises a temperature controller 25.

In the temperature controller 25, a pre-set temperature threshold may be stored. If the temperature of the plasma activated water 4 in the incubation tank 14 exceeds the pre-set threshold temperature, the heating and cooling device 23 lowers the temperature below the threshold, thus ensuring that temperature sensible compounds in the biomass 2 do not decompose.

Alternatively, the temperature control system 22 may be a closed-loop system with a reference temperature stored in the temperature controller 25. The temperature sensor 24 continuously measures the temperature of the plasma activated water 4 in the incubation tank 14 and transfers temperature data representative of the actual temperature of the plasma activated water 4 in the incubation tank 14 to the temperature controller 25. The temperature controller 25 compares the actual temperature of the plasma activated water 4 in the incubation tank 14 with the reference temperature. In case of a deviation, the temperature controller 25 sends a control signal to the heating and cooling device 23 for lowering/raising the temperature of the plasma activated water 4 in the incubation tank 14, thus controlling it to the reference temperature.

In the following, another exemplary embodiment of a system 1 for pre-treating biomass 2 for improving accessibility of a compound therefrom is disclosed with reference to Fig. 13. The same reference numerals will be used for elements having similar functions as the elements used in Fig. 12 with respect to the first embodiment. Further, only the differences between the second embodiment of Fig. 13 with respect to the first embodiment shown in Fig. 12 are described.

The system 1 of the second embodiment allows a continuous pre-treatment of biomass 2, in particular biomass that is resistant to cell disruption, for improving accessibility and the extraction of a compound, such as an intracellular compound therefrom.

The system of the second embodiment comprises a piping 26. A first section 27 of the piping 26 corresponds to the incubation container 13 of the plasma activated water stage 3. A second section 28 of the piping 26 corresponds to the electroporation container 10.

At the feeding end 29 of the first, plasma activated water section 27, biomass 2 is introduced from the biomass storage 30 and mixed with plasma activated water 4 from plasma activated water container 16. The biomass 2 and the plasma activated water 4 are both fed continuously to the feeding end 29 and are continuously mixed and transported through the piping 26 by means of transport devices 12 in the shown embodiment two pumps 31 and 32. One pump 31 is arranged in the first section 27, the other pump 32 is arranged in the second section 28 of the piping 26.

When passing through the first, plasma activated water section 27 of the piping 26, the biomass 2 is incubated in the plasma activated water 4. The incubation time may be determined by the length of the first piping section 27 as well as the flowrate of the pump 31.

The transfer from the downstream end 36 of the first section 27, opposite the feeding end 29, of the piping is depicted by an arrow on the right side of Fig. 13. The downstream end 36 is connected with the insertion end 37 of the second section 28 of the piping 26, indicated by the arrow on the left side of the second section 28 of the piping 26. In reality, piping 26 would of course be a continuous conduit.

In electric field stage 5, two ring electrodes 7, 8 are placed spaced apart from each other along the length of the piping 26 around the piping for generating an electric field within the piping 26 for applying the electric field, such as a pulse electric field, on the biomass transported by the pump through the second, electroporation section 28 of the piping 26.

This way, biomass 2 may be continuously processed by first incubating it in plasma activated water 4 in the plasma activated water stage 3 and subsequently applying an electric field in the electric field stage 5.

In order to provide a constant supply of plasma activated water 4, the plasma activated water generation device 15 of the second embodiment shown in Fig. 13 comprises a plasma activated water container 16, from which the plasma activated water 4 can be continuously fed to the feeding end 29 of the piping 26 wherein it is mixed with biomass 2.

For a continuous supply, the plasma activated water 4 removed from plasma activated water container 16 is replaced with water from a plasma activated water buffer 33 via a buffer line 34 and a buffer pump 35 arranged in the buffer line 34. A plasma generator 17 is arranged in the plasma activated water buffer 33. Water can be fed by a feed pump 38 from a water feed line 39 into the plasma activated water buffer 33, in which plasma may be generated using the plasma generator 17. The plasma activated water container 16 comprises a level sensor 40, such as for example an ultrasound level sensor. If the level of the plasma activated water 4 in the plasma activated water container 16 drop below a defined threshold level, the level sensor 40 may send a refill signal to activate the buffer pump 34 and transfer plasma activated water 4 held in the plasma activated water buffer 33 to refill the container 16. The plasma activated water buffer 33 may also comprise a level sensor 41. If the level of the plasma activated water 4 in the plasma activated water buffer 33 drop below a defined threshold level, the level sensor 41 may send a replenishment signal. The signal activates the feed pump 38 resulting in a transfer of water from the water feed line 39 refilling the buffer 33 and it also activates the plasma generator 17 thus producing more plasma activated water 4 in the buffer 33. This way a constant supply of plasma activated water in the plasma activated water container 16 is provided.

REFERENCE NUMERALS

| 1 | System |
|---|---|
| 2 | Biomass |
| 3 | Plasma activated water stage |
| 4 | Plasma activated water |
| 5 | Electric field stage |
| 6 | Capacitor |
| 7 | Electrode |
| 8 | Electrode |
| 9 | Pulse generator |
| 10 | Electroporation container |
| 11 | Transfer element |
| 12 | Transport device |

13 Incubation container
14 Incubation tank
15 Plasma activated water generation device
16 Plasma activated water container
17 Plasma generator
18 Mixer
19 Stirrer
20 Impeller
21 Impeller shaft
22 Temperature control system
23 Heating and cooling device
24 Temperature sensor
25 Temperature controller
26 Piping
27 First section
28 Second section
29 Feeding end
30 Biomass storage
31 Pump
32 Pump
33 Plasma activated water buffer
34 Buffer line
35 Buffer pump
36 Downstream end
37 Insertion end
38 Feed pump
39 Water feed line
40 Level sensor
41 Level sensor

**Claims**

1. Method of pre-treating biomass (2), in particular biomass (2) that is resistant to cell disruption, for improving the accessibility of a compound therefrom, comprising the step of:

   - incubating the biomass (2) in plasma activated water (4).

2. Method of pre-treating biomass (2) according to claim 1, wherein the biomass (2) is incubated at least 5 minutes, preferably 10 to 90 minutes, and most preferably 45 to 75 minutes.

3. Method of pre-treating biomass (2) according to claim 1 or 2, wherein the plasma activated water (4) has an acidic pH lower than 3.5, preferably lower than 3.0, and most preferably lower than 2.5.

4. Method of pre-treating biomass (2) according to any of claims 1 to 3, wherein the plasma activated water (4) has an oxidation reduction potential of at least 250 mV, preferably at least 350 mV, and most preferably above 450 mV.

5. Method of pre-treating biomass (2) according to any one of claims 1 to 4, wherein the plasma activated water (4) comprises a nitrate concentration of at least 40 mg/L, preferably of at least 150 mg/L, and most preferably of over 350 mg/L.

6. Method of pre-treating biomass (2) according to any one of claims 1 to 5, wherein the plasma activated water (4) comprises a nitrite concentration of at least 2 mg/L, preferably of over 6 mg/L.

7. Method of pre-treating biomass (2) according to any one of claims 1 to 6, comprising the further pre-treatment step of:

   - applying an electric field to the biomass (2).

8. Method of pre-treating biomass (2) according to claim 7, wherein a pulsed electric field is applied to the biomass (2).

9. Method of pre-treating biomass (2) according to any one of claims 7 and 8, wherein the energy input, while applying the electric field, is at least 10 kJ/kg, preferably at least 50 kJ/kg, and most preferably in the range of 50 to 200 kJ/kg.

10. Method of pre-treating biomass (2) according to any one of claims 7 to 9, wherein an electric field of 1 to 50 kV/cm, preferably of 5 to 20 kV/cm, and most preferably of 10 -15 kV/cm is applied, and/or where at least 10 pulses, preferably 10 to 300 pulses, and most preferably 40 to 80 pulses are applied.

11. Method of pre-treating biomass (2) according to any one of claims 7 to 10, wherein the electric field is applied before, while and/or after the incubation in plasma activated water (4), wherein preferably the biomass (2) is first incubated in plasma activated water (4) and subsequently an electric field is applied.

12. Method for extracting a compound from a biomass (2), in particular a biomass (2) that is resistant to cell disruption, comprising the steps of

   - pre-treating the biomass (2) according to any one of claims 1 to 11; and
   - extracting the compound from the pre-treated biomass (2).

13. Method of pre-treating biomass (2) according to any one of claims 1 to 11, or method for extracting a compound according to claim 12, wherein the biomass (2) is selected from the group comprising: human, animal, plant and microbial sources, preferably yeast, fungus, bacteria and spores, algae, macroalgae, microalgae, animal and/or plant-based raw material, non-edible seeds, and a biological waste material, like fermentation residues and waste products from food production.

14. Method of pre-treating biomass (2) according to any one of claims 1 to 11 and 13, or method for extracting a compound according to any one of claims 12 and 13, wherein the compound is selected from the group comprising: lipids, polyunsaturated fatty acids, eicosapentaenoic acid, docosahexaenoic acid, proteins, carbohydrates, vitamins, tocopherols, chlorophyll, phycocyanin, carotenoids, phenolic compounds, intracellular DNA/RNA, cell organelles, enzymes, and cell wall compounds, such as Glucans, $\beta$-(1,3)/(1,6)-Glucan, $\beta$-carotene, astaxanthin, zeaxanthin, sitosterol, microcolin, lutein, and phycobiliproteins.

15. System (1) for pre-treating biomass (2), in particular biomass (2) that is resistant to cell disruption, for improving the accessibility of a compound therefrom, comprising:

  - a plasma activated water stage (3) configured to incubate the biomass (2) in plasma activated water (4); and
  - an electric field stage (5) comprising at least one capacitor (6) for generating and applying an electric field to the biomass (2).

FIG. 1

EP 3 904 492 A1

FIG. 2

FIG. 3

FIG. 4

FIG. 5

FIG. 6

FIG. 7

FIG. 8

FIG. 9

FIG. 10

FIG. 11

FIG. 12

FIG. 13

Europäisches
Patentamt

European
Patent Office

Office européen
des brevets

# EUROPEAN SEARCH REPORT

Application Number

EP 20 17 2130

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>A | WO 2018/226313 A1 (GREENPATH IND LLC [US]) 13 December 2018 (2018-12-13)<br>* pages 1-6,10 *<br>* page 11 *<br>* claims 1,12,16,19; figures 1,2,5 * | 1-6,13, 15<br>7-12,14 | INV.<br>C12M1/00 |
| X | DATABASE WPI<br>Week 201743<br>Thomson Scientific, London, GB;<br>AN 2017-31297Y<br>XP002800328,<br>& CN 106 629 980 A (UNIV DALIAN MINZU) 10 May 2017 (2017-05-10)<br>* abstract * | 1-6,13, 15 | |
| A | WO 2014/055812 A1 (EP TECHNOLOGIES LLC [US]) 10 April 2014 (2014-04-10)<br>* paragraphs [0007], [0014] - [0018] * | 1,12,15 | |
| A | US 9 475 713 B2 (ZOLEZZI GARRETON ALFREDO [CL]; ADVANCED INNOVATION CENTER LLC [US]) 25 October 2016 (2016-10-25)<br>* column 3 - column 4 * | 1,12,15 | TECHNICAL FIELDS SEARCHED (IPC) |
| A | DATABASE COMPENDEX [Online]<br>ENGINEERING INFORMATION, INC., NEW YORK, NY, US;<br>2016,<br>COUSTETS M ET AL: "The use of pulsed electric fields for protein extraction from nanochloropsis and chlorella",<br>XP002800329,<br>Database accession no. E20154501513310<br>* abstract * | 1,12,15 | C12M |
| A,D | WO 2017/081677 A1 (RAMOT AT TEL-AVIV UNIV LTD [IL]) 18 May 2017 (2017-05-18)<br>* the whole document * | 1,12,15 | |

The present search report has been drawn up for all claims

| Place of search | Date of completion of the search | Examiner |
|---|---|---|
| Berlin | 14 September 2020 | Böhm, Ingo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

............................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 17 2130

This annex lists the patent family members relating to the patent documents cited in the above-mentioned European search report.
The members are as contained in the European Patent Office EDP file on
The European Patent Office is in no way liable for these particulars which are merely given for the purpose of information.

14-09-2020

| Patent document cited in search report | | | Publication date | Patent family member(s) | | | Publication date |
|---|---|---|---|---|---|---|---|
| WO 2018226313 | A1 | | 13-12-2018 | AU | 2018281812 | A1 | 30-01-2020 |
| | | | | CA | 3064831 | A1 | 13-12-2018 |
| | | | | EP | 3634618 | A1 | 15-04-2020 |
| | | | | US | 2020071199 | A1 | 05-03-2020 |
| | | | | WO | 2018226313 | A1 | 13-12-2018 |
| CN 106629980 | A | | 10-05-2017 | NONE | | | |
| WO 2014055812 | A1 | | 10-04-2014 | AU | 2013327002 | A1 | 14-05-2015 |
| | | | | BR | 112015007537 | A2 | 04-07-2017 |
| | | | | CA | 2887339 | A1 | 10-04-2014 |
| | | | | CN | 104837349 | A | 12-08-2015 |
| | | | | EP | 2903443 | A1 | 12-08-2015 |
| | | | | JP | 2015533828 | A | 26-11-2015 |
| | | | | KR | 20150079673 | A | 08-07-2015 |
| | | | | US | 2014100277 | A1 | 10-04-2014 |
| | | | | WO | 2014055812 | A1 | 10-04-2014 |
| US 9475713 | B2 | | 25-10-2016 | AU | 2011213979 | A1 | 25-10-2012 |
| | | | | CA | 2789402 | A1 | 18-08-2011 |
| | | | | CL | 2012002219 | A1 | 25-01-2013 |
| | | | | CN | 102870502 | A | 09-01-2013 |
| | | | | CO | 6592100 | A2 | 02-01-2013 |
| | | | | EA | 201201121 | A1 | 28-06-2013 |
| | | | | EP | 2534931 | A2 | 19-12-2012 |
| | | | | IL | 221362 | A | 29-05-2017 |
| | | | | IL | 249792 | A | 28-06-2018 |
| | | | | JP | 6243122 | B2 | 06-12-2017 |
| | | | | JP | 2013519503 | A | 30-05-2013 |
| | | | | JP | 2017018944 | A | 26-01-2017 |
| | | | | KR | 20120130768 | A | 03-12-2012 |
| | | | | MA | 34061 | B1 | 05-03-2013 |
| | | | | MX | 349617 | B | 04-08-2017 |
| | | | | NZ | 602420 | A | 26-06-2015 |
| | | | | SG | 184117 | A1 | 30-10-2012 |
| | | | | SG | 10201506138U | A | 29-09-2015 |
| | | | | US | 2011240567 | A1 | 06-10-2011 |
| | | | | US | 2016272518 | A1 | 22-09-2016 |
| | | | | WO | 2011098918 | A2 | 18-08-2011 |
| | | | | ZA | 201206804 | B | 28-05-2014 |
| WO 2017081677 | A1 | | 18-05-2017 | EP | 3374515 | A1 | 19-09-2018 |
| | | | | US | 2018223272 | A1 | 09-08-2018 |
| | | | | WO | 2017081677 | A1 | 18-05-2017 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019121630 A1

- WO 2017081677 A

**Non-patent literature cited in the description**

- **PARNIAKOV et al.** Pulsed electric field assisted extraction of nutritionally valuable compounds from microalgae Nannochloropsis spp. using the binary mixture of organic solvents and water. *Innovative Food Science and Emerging Technologies,* 2015, vol. 27, 79-85